Europäisches Patentamt

European Patent Office

Office européen des brevets

(11) Publication number: **0 163 406**
**B1**

(12) **EUROPEAN PATENT SPECIFICATION**

(45) Date of publication of patent specification: **09.01.91**

(21) Application number: **85302744.9**

(22) Date of filing: **18.04.85**

(51) Int. Cl.⁵: **C 12 N 15/62, C 12 N 15/67, C 12 P 19/34, C 12 N 1/20, C 12 P 21/02, C 07 K 7/40**

(54) **Novel polypeptide expression method.**

(30) Priority: **01.05.84 CA 453270**

(43) Date of publication of application:
**04.12.85 Bulletin 85/49**

(45) Publication of the grant of the patent:
**09.01.91 Bulletin 91/02**

(84) Designated Contracting States:
**BE CH DE FR GB IT LI NL SE**

(56) References cited:
**EP-A- 154 576**
**EP-A-0 040 466**
**US-A-4 342 832**
**US-A-4 366 246**

(73) Proprietor: **Canadian Patents and Development Limited Société Canadienne des Brevets et d'Exploitation Limitée**
**275 Slater Street**
**Ottawa Ontario, K1A 0R3 (CA)**

(72) Inventor: **Garvin, Robert T.**
**33 DeForest**
**Toronto Ontario M6S 1J1 (CA)**
Inventor: **Shen, Shi-Hsiang**
**15 Rockford Drive Apt. 1105**
**Willowdale Ontario M2R 3A3 (CA)**
Inventor: **James, Eric**
**3303 Don Mills Road Apt. 2903**
**Willowdale Ontario M2J 3A3 (CA)**

(74) Representative: **Burford, Anthony Frederick et al**
**W.H. Beck, Greener & Co. 7 Stone Buildings Lincoln's Inn**
**London WC2A 3SZ (GB)**

Courier Press, Leamington Spa, England.

# EP 0 163 406 B1

**Description**

Bacteria, in common with mammalian and other eucaryotic cells, possess a proteolytic system for the selective degradation of abnormal or no longer needed polypeptides. In the natural course of events, abnormalities may arise by either mutation or biosynthetic error. Alternatively, a normal polypeptide may be needed in the cell only at a specific time to carry out a specific vital function while, at any other time, the presence of that polypeptide is deleterious to the cell, so that, once the polypeptide has carried out its function, it is selectively destroyed. These operations imply a complex network of interacting regulatory and structural elements, and, indeed, in *Escherichia coli*, the best studied bacterial system, eight different endoproteases have so far been described which function, individually or in concert, in this selective degradation system.

The introduction of foreign genes into a microorganism often has inadvertently activated this proteolytic system, thereby inhibiting the large scale production of a desired gene product by the practice of genetic engineering. This problem is particularly acute when genes coding for eucaryotic peptides are introduced into the bacteria. The strategy for circumventing this problem has been to fuse the nucleotide sequence coding for the desired gene product to at least part of a structural gene, or carrier protein gene, whose product is known to be constitutively stable.

The tandem arrangement of desired gene product and carrier protein results in the expression from the organism of a hybrid polypeptide which is stable, provided that the carrier protein is large enough to identify the entire hybrid polypeptide as both native and constitutively stable. The prior art difficulty of direct expression of eucaryotic polypeptides and the necessity to use a carrier protein to achieve expression is well recognized and has been described in a number of scientific papers and published patent applications and patents, for example, in U.S. Patent No. 4,342,832. A typical patent which describes the use of this technique, for the production of somatostatin, is U.S. Patent No. 4,366,246.

A disadvantage to this approach, however, is that the desired product constitutes only a small portion of the hybrid polypeptide, thereby decreasing significantly the theoretical yield, and necessitating further processing to split off the native carrier protein and to purify the desired polypeptide product. Efforts to decrease the native carrier moiety to a small portion of the hybrid product in an attempt to improve the yield, however, render the expressed hybrid polypeptide unstable.

The symbols, abbreviations and some of the terms used herein have the following meanings:

DNA—deoxyribonucleic acid
A—Adenine
T—Thymine
G—Guanine
C—Cytosine
Tris—2-Amino-2-hydroxy-ethyl-1,3-propane diol
EDTA—Ethylene diamine tetraacetic acid
ATP—Adenosine triphosphate
TTP—Thymidine triphosphate
GTP—Guanosine triphosphate
T4—Bacteriophage specific to *E. coli*
Nucleotide—Nucleoside phosphate
Codon—Three nucleotide sequences specifying a particular amino acid
Oligonucleotide—Polymer consisting of a number of nucleotides
Plasmid—Self-replicating, extrachromosomal genetic element

In accordance with the present invention, it has now surprisingly been found that this prior art problem can be satisfactorily overcome and the natural, selective proteolytic mechanisms of the microorganisms can be circumvented to achieve high yield production of a desired eucaryotic polypeptide. This is achieved by tandemly-linking a plurality of polypeptide genes through selectively cleavable amino acid sequences, incorporating the resulting DNA construct into a cloning vehicle, and transforming a microorganism with the cloning vehicle.

In accordance with the present invention, therefore, there is provided a DNA fragment composed of repeating units coding for a desired product of known amino acid sequence tandemly-linked by oligonucleotides coding for a selectively cleavable amino acid sequence and having no codons for operative combinations of stop and start signals between the ends of said fragment, whereby the fragment is expressible in a suitable vehicle to produce a polypeptide composed of tandemly-linked repeating units of the said desired product.

By providing a DNA fragment composed of repeating units coding for a desired product of known amino acid sequence and separated by oligonucleotides coding for selectively cleavable amino acid units, a replicable cloning vehicle can be provided which is capable of stably expressing a product in a microbial organism. Once the product has been expressed during growth of the microorganism, it may be isolated from the cell culture, and the desired polypeptide product is obtained from the isolated product by cleaving the repeating units at the cleaving sites to form a plurality of individual units. Since multiple copies of the desired polypeptide product are expressed from the microorganism, the yield of product is considerably enhanced with respect to the prior art expression procedure discussed above.

2

The present invention has general applicability to the enhancement of stability of polypeptides in microorganisms and may be used to produce a wide variety of polypeptide products, comprising hormone polypeptides, including human and animal source polypeptides, for example, human proinsulin, human growth hormone, somatostatin and human interferon, and viral proteins for use in vaccines.

Essential to the present invention is the formation of a DNA fragment which contains at least two units each coding for a desired product of known amino acid, sequence, separated by a joining unit whose product is selectively cleaved. The number of repeating units which is required to achieve stability depends on the desired product, while the nature of the joining unit and the cleaving agent used for cleaving also depend on the desired product.

The term "stability" as used herein with respect to the expression products refers to a material that has a half-life of sufficient duration to enable extraction thereof to be effected. It is preferred to provide by this invention products having a half-life greater than about 200 minutes.

The specific embodiment of the invention relates to the expression of human proinsulin, i.e. the precursor of insulin containing the C-chain in addition to the A and B chains, but the principles thereof are widely applicable to any polypeptide, as discussed above.

In the following description, reference is made to the accompanying drawings, in which:

Figure 1 is a schematic illustration of the formation of recombinant plasmids in accordance with this invention;

Figure 2 is a derivation and restriction site map of plasmid plac 239-3PI;

Figure 3 is a derivation and restriction site map of plasmid pTac/5PI;

Figure 4 illustrates the sequence of steps to form plasmid pAT/PIR;

Figure 5 is a derivation and restriction site map of plasmid pAT/PIR;

Figure 6 illustrates the sequence of steps to form plasmid plac 504/PI, and formation of plac 239-PI and pTac/PI therefrom;

Figure 7 is a derivation and restriction site map of plasmid plac 504/PI;

Figure 8 illustrates formation of plasmid plac 239-PI from plac 504/PI; and

Figures 9, 10 and 11 are photographs of electrophoresis experiments conducted on various products produced in accordance with this invention.

In the utilization of the present invention to effect the production of human proinsulin in accordance with the preferred embodiment hereof, a DNA fragment is formed containing a plurality of sequences coding for human proinsulin separated by oligonucleotide sequences coding for selectively-cleavable units, which, in the final product, permit ready separation of the individual proinsulin peptides one from another. Sequences coding for operative combinations of stop and start signals are absent from the fragment between its ends. The DNA fragment is inserted into a replicable cloning vehicle which is capable of expressing the fragment in a microbial organism.

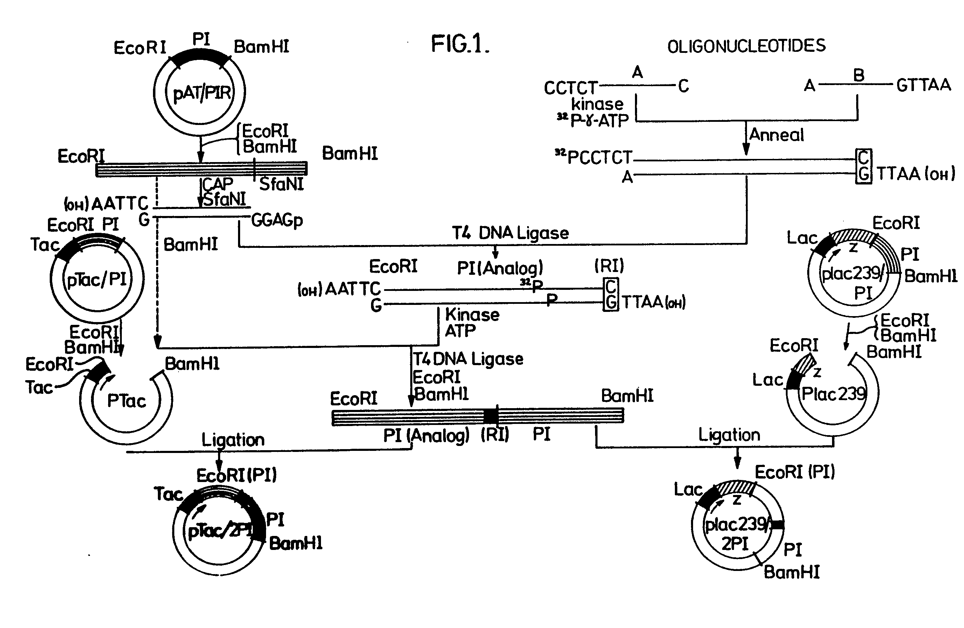

The preferred method of constructing the multiple proinsulin gene and inserting the same into a cloning vehicle, in both a fused and an unfused configuration, is illustrated in Figure 1. In the fused configuration, the human proinsulin coding sequence is joined to the 3'-side of a fragment containing the lac promoter and encoding the first 80 amino acids of the N-terminus of β-galactosidase. In the unfused configuration, the proinsulin coding sequence is linked directly to a fragment containing the Tac promoter, followed by a bacterial Shine-Delgarno sequence. It has been found that the yield of expressed polypeptide, in terms of grams per cell of microorganism, is significantly greater in the case of the fused configuration, as compared with the unfused configuration.

In both configurations, the single gene human proinsulin is too unstable to be isolated as a product from the microorganism but, as additional human proinsulin coding sequences are added to the genetic construct by the method of Figure 1, the resultant multiple domain polypeptide becomes increasingly stable, approaching normal stability at three tandemly-linked proinsulin domains in the unfused configuration, and at two tandemly-linked proinsulin domains in the fused configuration. As increasing numbers of proinsulin coding sequences are tandemly linked, a maximum number of coding sequences is reached, beyond which the yield of polymer declines. For example, increasing the number of tandemly-linked units in the unfused configuration for proinsulin beyond six, results in a decreased amount of polymeric product being produced by the cell. It is preferred to utilize 4 to 5 proinsulin coding sequences in the unfused configuration and 3 to 4 proinsulin coding sequences in the fused configuration.

The oligonucleotide sequence for the linking unit or domain junction in the embodiment illustrated in Figure 1 is represented as follows:

Proinsulin gene-Arg Arg Asn Ser Met-Proinsulin gene

AGG CGC AAT TCT ATG

The amino acid domain junction is easily cleavable, by reaction with cyanogen bromide, into single proinsulin peptides. This domain junction and its manner of cleaving are but one example of a variety of linking units and modes of cleaving which may be adopted.

For those polypeptides which contain no methionine group, cyanogen bromide cleavage may be used to produce the native material directly, provided that a methionine codon constitutes the peptide coding

3

junction, while for those polypeptides which do contain the methionine group, the design of a particular amino acid sequence positioned between desired polypeptide segments to provide an easily-cleavable site may be effected by genetic engineering methods. For example, polypeptides which do not contain an aspartyl-proline bond may be engineered to contain such a bond between each unit of the multi-coding sequence construct, so that recovery of the native material from the isolated multi-domain polypeptide may be achieved following cleavage by acids, as described by Jauregui-Adell and Marti, Anal. Biochem. *69*, 468 (1975).

In general, enzymatic methods or chemical reactions which are amino acid residue-sequence specific may be used to effect cleavage of linking groups between the desired polypeptide genes.

The authentic human proinsulin molecule contains no methionine residues, so that the cyanogen bromide treatment cleaves a multi-domain proinsulin molecule into several proinsulin analog moieties along with the C-terminal authentic proinsulin unit. This results from the designed placement of methionine codons at the 5'-end of each of the proinsulin coding sequence units.

The individual proinsulin units derived in this manner may be further processed into authentic human insulin by digestion with trypsin and carboxypeptidase B, or by any other convenient procedure.

The formation of a recombinant plasmid containing an oligonucleotide sequence coding for multiple copies of human proinsulin leads to a considerable increase in yield of proinsulin and proinsulin analogs, when compared with the expression in the prior art of the hybrid molecule containing the β-galactosidase gene.

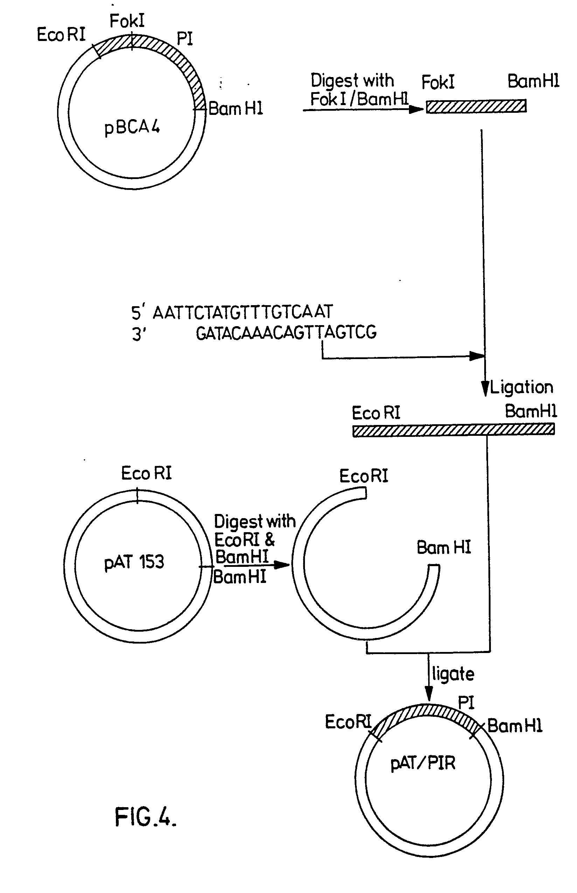

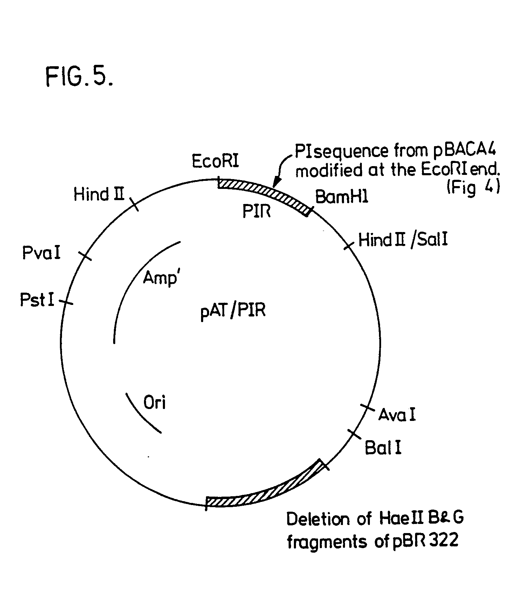

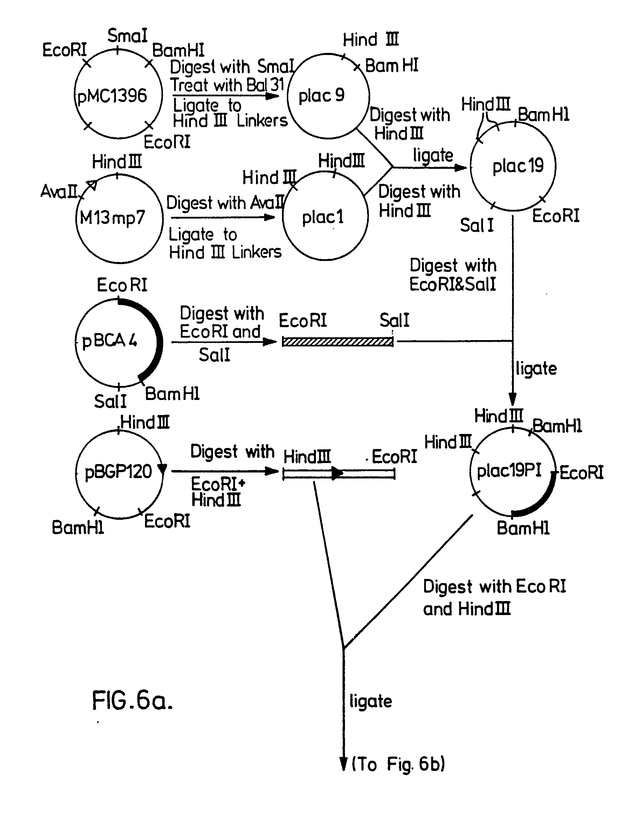

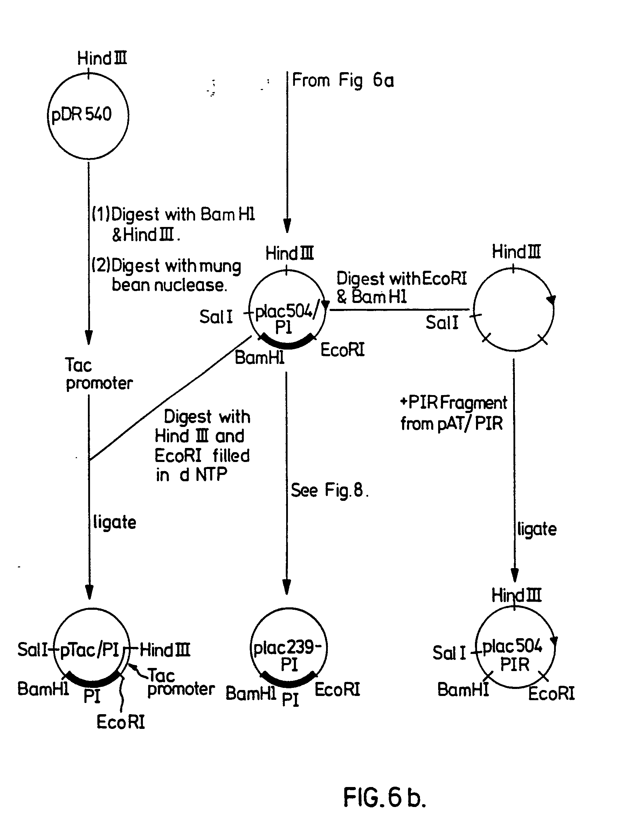

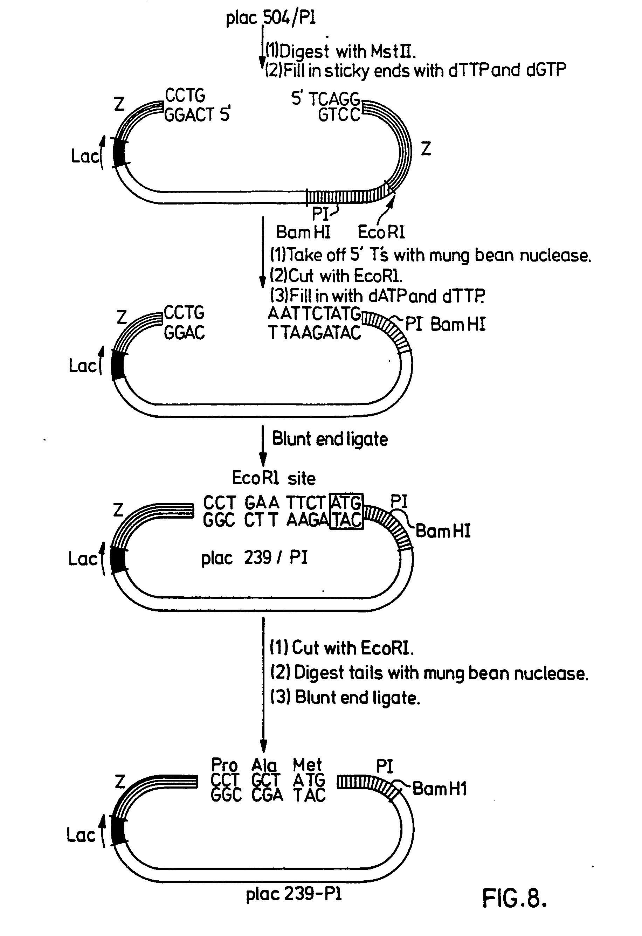

As seen in Figure 1, the preferred sequence to form the novel plasmids p*Tac*/2PI and plac 239/2PI, and other multiple PI unit plasmids especially the expression plasmids p*Tac*/5PI and plac 239/3PI which is further engineered as illustrated in Figure 8 to form the preferred expression plasmid plac-3P1, starts from the plasmid pAT/PIR which contains an oligonucleotide sequence between the EcoRI and BamHI restriction sites coding for human proinsulin. The derivation from the known plasmids pBCA4 of the plasmid pAT/PIR, which is also a novel plasmid, is illustrated in Figure 4. The p*Tac* and plac 239 fragments are derived from plasmids p*Tac*/PI and plac 239/PI respectively, which in turn are both derived from another novel plasmid, plac 504/PI, as shown in Figure 6. The derivation of plasmid plac 504/PI from known plasmids is also shown in Figure 6.

It is not essential to obtain the p*Tac* and plac vectors from the plasmids p*Tac*/PI and plac/PI i.e. plasmids containing a single copy of the proinsulin gene. They may be derived from any other convenient plasmid which contains the *Tac* and lac promotor respectively, and which can be cleaved at EcoRI and Bam HI sites to form the vector for ligation with the multiple proinsulin gene fragment. Similarly, it is not essential to form the plasmid plac 504/PI, i.e. also containing the single copy of the proinsulin gene, as an intermediate, but any other convenient intermediate plasmid may be employed. The particular single copy proinsulin gene plasmids here embodied were formed by the inventors during the experimentation which led to this invention in an attempt to provide constructs from which proinsulin would be stably expressed. As noted earlier, this was not found to be possible with the single copy gene.

Specific experimental details for the formation of the various plasmids set forth in the drawings and disclosed above are discussed in the Examples below.

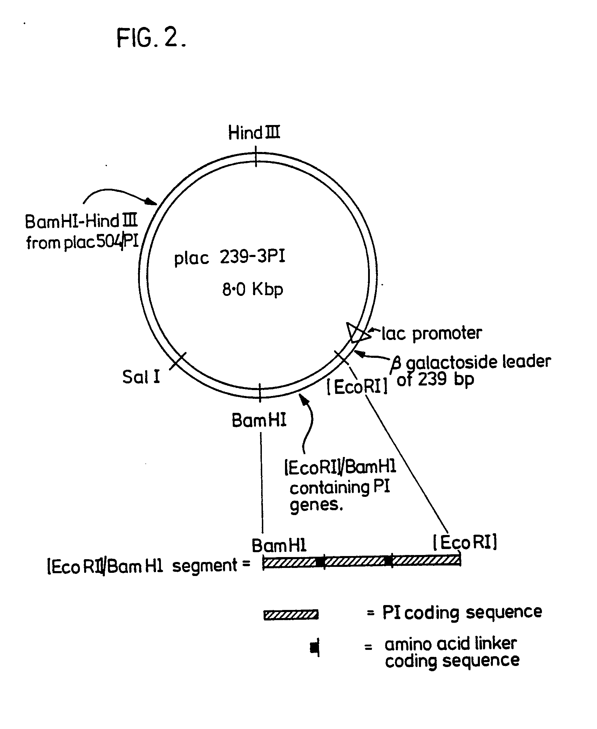

Detailed derivations and major restriction site maps for the novel plasmids plac 239-3PI, p*Tac*/5PI, pAT/PIR and plac 504/PI appear as Figures 2, 3, 5 and 7 respectively. *E. coli* JM103 modified by each of the plasmids plac 239-3PI and p*Tac*/5PI, have been deposited on July 6, 1984 with the American Type Culture Collection located at Rockville, Maryland, U.S.A., and have been accorded the following accession Nos.:

Organism *E. coli*/plasmid plac 239-3PI—ATCC 39760
Organism *E. coli*/plasmid p*Tac*/5PI—ATCC 39759.
The invention is illustrated further by the following Examples.

Example 1
This Example describes the formation of novel plasmids containing multiple units of the human proinsulin gene.

(a) Preparation of pAT/PIR
The plasmid pAT/PIR was prepared following the sequence outlined in Figure 4 and the restriction map for the plasmid appears as Figure 5. The starting plasmid pBCA4 contains a nucleotide sequence coding for human proinsulin between its EcoRI and BamHI restriction sites. However, if the EcoRI-BamHI fragment of this plasmid is used directly, the wrong reading frame results and hence must be modified as described below. Plasmid pBCA4 is described in Narang et al, Gene, *17*, 279 (1982) and was obtained from Professor R. Wu, an author of that paper of Cornell University, Ithaca, N.Y., U.S.A.

The plasmid pBCA4 was cut with FokI and BamHI and the FokI-BamHI fragment was isolated. Two synthetic oligonucleotides were created and ligated with the FokI-BamHI fragment to extend the 5'-end of the PI sequence from FokI to EcoRI, the altered EcoRI-ended PI sequences being designated PIR. The synthetic nucleotide sequences were generated chemically and comprised the sequences:

5'  AATTCTATGTTTGTCAAT        3'

3'        GATACAAACAGTTAGTCG  5'

The starting plasmid pAT153 is a high copy variant of the well-known plasmid pBR322 with the so-called "poison" sequences removed and is described by Twigg et al, Nature, 283, 216 (1980). The plasmid pAT153 was obtained from Professor O. Smithies, Dept. of Genetics, University of Wisconsin—Madison, Madison, Wisconsin, U.S.A. The plasmid pAT153 was cut with EcoRI and BamHI and the vector isolated. The isolated vector was ligated to the EcoRI-BamHI (PIR) fragment, to form the new plasmid pAT/PIR.

(b) Preparation of multiple copies of proinsulin gene

The sequence of operations required to form multiple copies of the proinsulin gene is outlined in Figure 1. Synthetic oligonucleotides (A) and (B) were prepared chemically to provide the following sequences:

(A)  5'  CCTCTACCAGCTGGAGAACTACTGCAACAGGCGC        3'

(B)  3'        ATGGTCGACCTCTTGATGACGTTGTCCGCGTTAA  5'

200 p.moles of oligonucleotide B and 200 p.moles of oligonucleotide A having its 5'-end labelled with $^{32}$P-ATP, were mixed in 30 ul of 66 mM Tris-HCl (pH 7.6), 6.6 mM $MgCl_2$ and 10 mM dithiothreitol, and heated at 80°C for 15 minutes to anneal the oligonucleotides to form a part of the human proinsulin coding sequence beginning at the SfaNI cut site near the 3'-end of the coding sequence and terminating with the last proinsulin codon (AAC), followed by the additional sequences:

5'  AGG CGC        3'

3'  TCC GCG TTAA  5'

These additional sequences of the synthetic oligonucleotides encode two arginine residues and provides a sticky end for ligation to an EcoRI sticky end. The ligation destroys the EcoRI site because of the CG base pair (boxed in Figure 1).

10 μg of pAT/PIR, prepared as described in (a) above, was digssted for 1 hour at 37°C with 40 units each of EcoRI and BamHI in 40 μl of digestion buffer. The digested DNA was dephosphorylated by digestion at 37°C for 30 minutes with 4 units of calf intestine alkaline phosphatase in 200 mM Tris-HCl, pH 8.0. The proinsulin gene fragment (PIR) was isolated from 1% agarose gel by phenol extraction. The isolated fragment was further digested for 3 hours at 37°C with 6 units of SfaNI in 40 μl of digestion buffer and the EcoRI-SfaNI fragment of PIR was isolated.

100 p.moles of the annealed oligonucleotides (A) and (B) and approximately 4 p.moles of the isolated PIR EcoRI-SfaNI fragment were ligated for 14 hours at 15°C in 30 μl of 66 mM Tris-HCl (pH 7.6), 6.6 mM $MgCl_2$, 10 mM dithiothreitol and 1 mM ATP using 20 units of T4 DNA ligase. As a result of dephosphorlyation of the proinsulin gene fragment prior to SfaNI cleavage, and phosphorlyation of only oligonucleotide (A), as described above, the reaction was unidirectional, resulting in ligation of the proinsulin gene fragment PIR to the annealed oligonucleotide. The ligation reaction product was electrophoresed on 1.5% agarose gel and the resultant proinsulin gene analog construct (PI Analog) was isolated from the gel.

The isolated proinsulin gene analog sequence (approximately 3 p.moles) was phosphorylated with ATP and T4 polynucleotide kinase. 10 μg of plasmid pAT/PIR, prepared as described in (a) above, was digested for 1 hour at 37°C with 40 units each of EcoRI and BamHI. Approximately 3 p.moles of the PIR proinsulin gene sequence were isolated from agarose gel. The isolated proinsulin gene sequence and phosphorlyated proinsulin gene analog sequence were ligated at 37°C for 3 hours in 30 μl of ligation buffer containing 20 units of T4 ligase, 40 units of EcoRI and BamHI.

The ligation mixture was run on 1.0% agarose gel and, after autoradiography, the dimer, i.e. two proinsulin coding sequences, and trimer, i.e. three proinsulin coding sequences, were isolated from the gel by phenol extraction. The tetramer, pentamer and hexamer, i.e. four, five and six proinsulin coding sequences respectively, were obtained by further ligating the isolated fragments with additional proinsulin gene analogs following the general procedure described above.

(c) Preparation of pTac and plac 239 recombinants containing multiple PI units

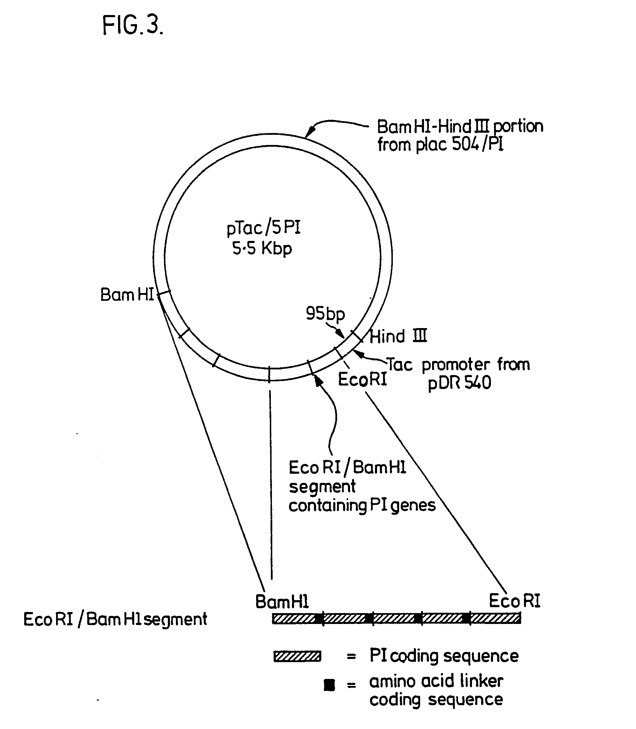

The various PI fragment constructs, containing from two proinsulin coding sequences up to six proinsulin coding sequences, were inserted into the vectors pTac and plac 239 by ligation, as generally described in (b) above. The pTac and plac 239 vectors were formed by digestion with EcoRI and BamHI of pTac/PI and plac 239/PI respectively, which, in turn, were formed following the procedures which are outlined below in sections (d) and (e). By inserting a proinsulin gene trimer into the plac 239 vector and a proinsulin gene pentamer into the pTac vector, there were obtained the fused and unfused multiple proinsulin gene plasmids plac 239/3PI and pTac/5PI respectively. The derivation and main restriction site map for the latter new plasmid appears as Figure 3.

In similar manner, other multiple proinsulin gene plasmids, including plac 239/4PI, plac 239/5PI, pTac/3PI, pTac/4PI, and pTac/6PI were constructed from the various multimer proinsulin genes and the plac 239 and pTac vectors. The various constructs, containing from 1 to 6 copies of the proinsulin coding sequence, were confirmed by restriction mapping and DNA sequence analysis.

(d) Preparation of plac 239-PI

The derivation of plasmid plac 239-PI is illustrated in Figure 6 and was prepared from plasmid plac 504/PI, as shown in detail in Figure 8. The formation of plasmid plac 504/PI is described in more detail below in section (f). The plasmid plac 504/PI has a lac promoter, the β-galactosidase gene (z-gene) complete to the EcoRI site near the carboxy terminal of the gene with the human proinsulin gene insert of plasmid pBCA4 fused at this EcoRI site, so as to remain in reading frame, such that the translation of the z-gene continues directly to code for proinsulin.

Referring to Figure 8, plasmid plac 504/PI was digested with MstII and the resulting sticky ends were filled in with dTTP and dGTP. The 5'-T's were then removed by digestion with mung bean nuclease, the fragment cut with EcoRI to remove the Mst II/EcoRI segment of the z-gene, the sticky ends of EcoRI were filled in with dATP and dTTP, and the plasmid was blunt-end ligated to establish a new EcoRI site, the resulting plasmid being designated as plac 239/PI. The plasmid was cut with EcoRI at the new site, the fragment EcoRI tails were digested with mung bean nuclease, and the resulting fragment blunt-end ligated to form the plasmid plac 239-PI in which the correct PI reading frame was restored. In order to restore the correct PI reading frame in other fused plasmids, for example, plac 239/3PI, the EcoRI tails should also be removed by the same procedure described above, to form the corresponding expression plasmid plac 239-3PI. The derivation and main restriction site map for plac-3PI appears as Figure 2.

(e) Preparation of pTac/PI

The preparation of plasmid pTac/PI is illustrated in Figure 6 and was effected from plac 504/PI and from plasmid pDR540. The plasmid pDR540 is described by Russell et al in Gene, Vol. 20, 231 (1982) and has the following features: a HindIII site upstream from the promoter, a −35 region from trp, the −10 operator, Shine-Delgarno, and Shine-Delgarno-to-start spacing regions of lac, with a BamHI site positioned at the start. The plasmid pDR540, purchased from P-L Biochemicals, Milwaukee, Wisconsin, U.S.A., was cut with BamHI and HindIII, the sticky ends removed with mung bean nuclease and the isolated Tac promoter was inserted by blunt-end ligation into the isolated HindIII-EcoRI fragment of plasmid plac 504/PI which contains the PI gene, by first digesting plasmid plac/PI with HindIII and EcoRI and then filling in the ends with DNA polymerase I. In this way, the Tac promoter of the plasmid pDR540 is substituted for the wild-type lac promoter and the z-gene portion of plac 504/PI, thereby preserving the sequence, spacing and properties of the hybrid promoter, but with an EcoRI sequence approriately located.

(f) Preparation of plac 504/PI

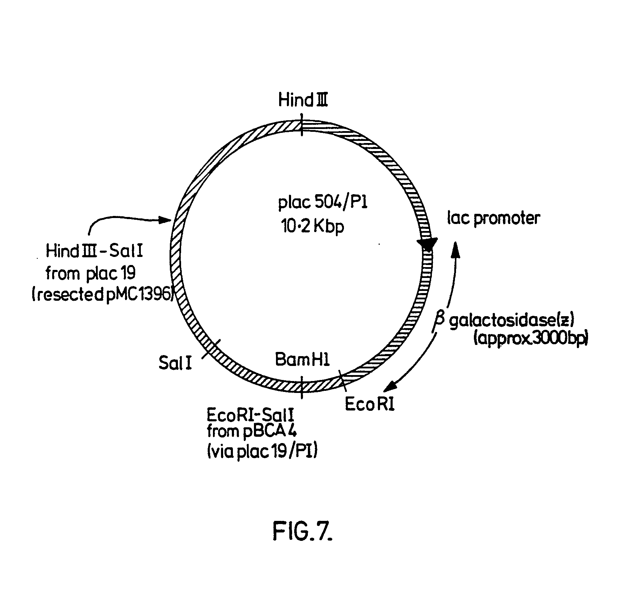

Plasmid plac 504/PI was prepared following the sequence of steps set forth in Figure 6 and the derivation and main restriction sites for the plasmid are illustrated in Figure 7.

The initial step in the formation of plasmid plac 504/PI was the formation of plasmid plac 19 from known plasmids pMC1396 and M13mp7. The plasmid pMC1396 is described by Casadaban et al in J. Bact., Vol. 143, 971, (1980) while the plasmid M13mp7 is described by Messing et al in Nucleic Acids Research, Vol. 9, 309 (1981). Plasmid M13mp7 was purchased from BRL Inc., Gaithersburg, MO, U.S.A. and plasmid pMC1396 was a gift of M. Casadaban of Cambridge, MA, U.S.A.

Plasmid pMC1396 was digested with SmaI, treated with Bal 31 and ligated with HindIII linkers to form plasmid plac 9. Plasmid pM13mp7 was digested with AvaII and ligated to a HindIII linker to form plasmid plac 1. Both plasmid plac 9 and plac 1 were digested with HindIII and ligated to form the plac 19 plasmid.

Plasmid pBCA4, containing the sequence coding for human proinsulin, was digested with EcoRI and SalI and the EcoRI—SalI fragment was isolated. Plasmid plac 19 was digested with EcoRI and SalI and the resulting vector was ligated with the EcoRI-SalI fragment of plasmid pBCA 4 to form plasmid plac 19/PI.

Plasmid pBGP120 contains the lac promoter and is described in Polisky et al, Proc. Nat. Acad. Sci. Vol. 73, 3900 (1976). This plasmid obtained from Dr. R. Wu of Cornell University, Ithaca, N.Y., U.S.A., was digested with EcoRI and HindIII and the HindIII-EcoRI fragment containing the lac promoter was separated. Following digestion of the plac19/PI plasmid with EcoRI and HindIII, the resulting vector was ligated with the HindIII-EcoRI fragment of plasmic pBGP120 to obtain the plasmid plac 504/PI.

Example 2

This Example illustrates the preparation of converted microorganisms from the novel plasmids containing multiple proinsulin genes and expression of the polyproinsulin polypeptide.

(a) Transformation and growth of cells

The novel plasmids plac 239-nPI, where n is 2 to 5, and pTac/mPI, where m is 3 to 6, were used to transform the strain of E. coli known as JM103. This strain is described by Messing et al in Nucleic Acids Res., 9, 309 (1981) and is characterized by the genotype (lac pro), thi, strA, supE, endA, sbcB, hsdR, F traD36, proAB, lacl$^q$, Z M15. The use of this E. coli strain was for convenience only and other E. coli strains and indeed other microorganisms could have been used.

Transformed strains were grown in YT medium containing 50 µg/ml of ampicillin. YT medium contains, per litre, 8 g tryptone, 5 g yeast extract, 5 g NaCl, and 1 ml 1N NaOH (to pH 7.0 to 7.2). In order to induce the expression of the polyproinsulin polypeptide from the plasmid, isopropyl-B-D-thiogalactoside (IPTG) was added to provide a final concentration of 1 mM when the cell density had reached an optical density of 0.1, measured at a wavelength of 560 nm.

(b) Recovery of expressed polypeptide

After induction by IPTG, cell growth was continued for about 8 to 12 hours. The cells were harvested by centrifugation, washed with phosphate-buffered saline, and either analyzed directly by SDS-PAGE (sodium dodecyl sulphate-polyacrylamide gel electropheresis), as described by Laemmli in Nature, 227, 680 (1970), or further purified. Such further purification was effected by suspending washed cells from 3 ml of culture in 1.0 ml of T buffer (i.e. 50 mM Tris HCl at pH 7.9 containing 25% sucrose, 1% Nonidet-P40, 0.5% sodium deoxychlolate and 5 mM EDTA) and subjecting the cells to sonic disruption by two 60 second bursts at a wavelength of 12 µm at 0°C. The sonicated material was centrifuged for 10 minutes in the Eppendorf microcentrifuge at 4°C. The centrifuged pellets were dissolved in the sample buffer and analyzed by SDS-PAGE.

(c) Analysis of expression from multiple PI gene constructs

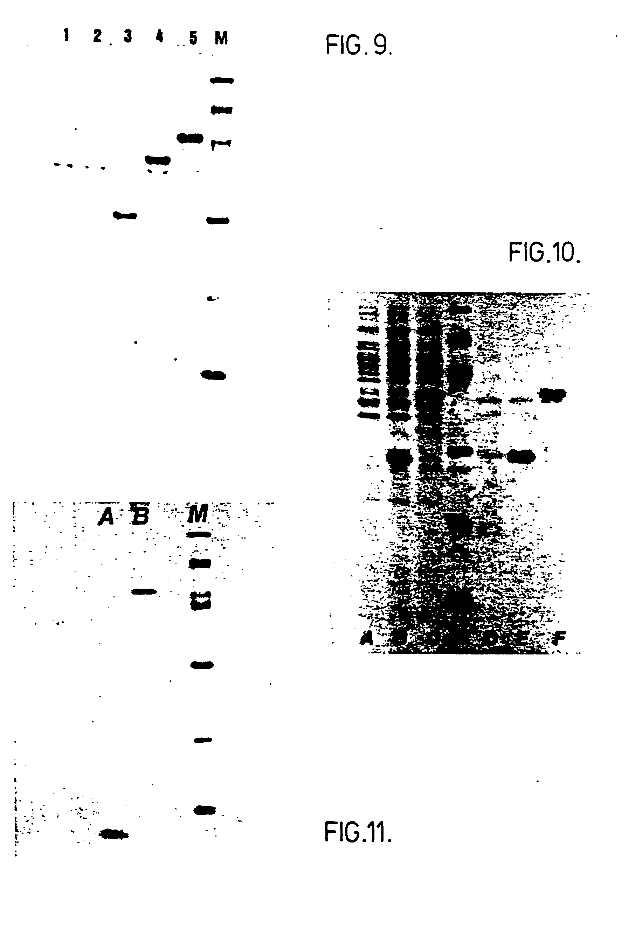

Electrophoretic analysis of multiply-expressed proinsulin polypeptides in the unfused configuration is shown in Figure 9. Proteins, partially purified as described above, were analyzed by 15% SDS-PAGE. Lanes 1 to 5 represent the products equivalent to 500 µl of original culture of JM103 cells harbouring the plasmids pTac/PI, pTac/2PI, pTac/3PI, pTac/4PI and pTac/5PI, while lane M contains protein molecular weight markers.

Electrophoretic analysis of multiply-expressed proinsulin polypeptide in the fused configuration is shown in Figure 10. Lanes A, B and C represent the total cell protein equivalent to 150 µl of original culture of JM103 cells harbouring the plasmids plac 239-PI, plac 239-2PI and plac 239-3PI while lanes D, E and F were the same products as lanes A, B and C, except that the products were partially purified, as described above. Lane M contains protein molecular weight markers.

The amount of product obtained per cell of microorganism was not determined quantitatively but was qualitatively observed to be significantly greater for the multiple gene fragments when compared with that known to be typically obtained when expressing hybrid polypeptides in the prior art.

Example 3

This Example illustrates the isolation and purification of polypeptide fractions and the formation of insulin therefrom.

(a) Recovery of proinsulin

About 7 g of cells were suspended in T buffer using 10 ml/g and were sonically disrupted at 0°C using two 90 second bursts at a wavelength of 18 µm. Following centrifugation at 12,000 g for 10 minutes, the pellet was suspended in 25 mM Tris-HCl, 2 M NaCl, 4 M urea at pH 8.4, using 5 ml/g of cells, resonicated and recentrifuged at 12,000 g. In order to solubilize the polyproinsulin, the pellet was resuspended in GDFT buffer (7 M guanidinium chloride, 100 mM sodium formate, pH 3.0) using 5 ml/g cells and gently stirred for 1 hour. The polyproinsulin contained in the GDFT buffer was precipitated by dilution into 6 volumes of ice-cold water, and collected by low speed centrifugation at 5,000 g. The resultant pellet contained essentially pure proinsulin polypeptide polymer, as determined by SDS-PAGE (see Figure 11, Lane B).

Purified proinsulin polymer was dissolved in 70% formic acid using 10 ml/2 g cell equivalents, digested with cyanogen bromide at 50 mg per mg of protein for 35 hours at room temperature, and the product analyzed by SDS-PAGE. Lane A in Figure 11 shows the cleaved product of the five tandem proinsulin polypeptide. Lane M contains protein molecular weight markers. A Western blot of the SDS-PAGE gel of Lanes A and B showed the human proinsulin-sized material to be reactive with porcine anti-proinsulin sera.

(b) Production of insulin

Recovered cyanogen bromide digest product was sulfitolyzed by dissolving the material in GE buffer, containing 6 M guanidinium HCl and 0.2 M ethanolamine (pH 9), at 10 mg/ml, adding $Na_2SO_3$ and $Na_2S_4O_6$ to concentrations of 0.4 M and 0.8 M respectively, and allowing the reaction to occur for 2 hours at room temperature. The sulfitolyzed product was precipitated by dialysis against 10 mM ammonium acetate (pH 4.5). The precipitate was dissolved at 4 mg/ml in TF buffer, containing 25% formamide and 50 mM Tris-HCl (pH 8.1) and the solution loaded onto a DEAE cellulose® column in the ratio of 200 mg per 2.5×10 cm

column. The products were differentially eluted by sodium chloride during application of a 0 to 500 mM linear NaCl gradient.

The sulfitolyzed biosynthetic human proinsulin (BHPI-SSO$_3$) was readily identifiable by HPLC-analysis of the salt gradient elution profile. BHPI-SSO$_3$ was collected and dialyzed at pH 4.5 to remove formamide and to precipitate the product. The pure BHPI-SSO$_3$ precipitate was dissolved in RH buffer, containing 50 mM glycine and 5 mM EDTA (pH 10.5), at 0.5 mg/ml and cooled to −2°C. Ethanethiol was added to 800 μm and allowed to react for 24 hours to effect refolding of the molecule. The reaction was stopped by adjusting the pH to 7 with HCl and the reaction product was analyzed. Refolded biosynthetic human proinsulin (BHPI) constituted the monomeric component of the reaction product.

The BHPI was dissolved in TC buffer, containing 100 mM Tris-HCl (pH 7.6) and 10 mM CaCl$_2$, at 2 mg/ml, 10 μg/ml of trypsin and 40 μg/ml of carboxypeptidase B were added, and the mixture was allowed to react for 30 minutes at room temperature. The reaction was stopped by adding acetic acid to 1% and biosynthetic human insulin (BHI) was recovered by precipitation. The recovered yield was 98% of theoretical, based on the amount of BHPI used.

The BHI was considered authentic on the basis of HPLC analysis, amino acid composition, amino acid sequencing and biological activity. Human proinsulin in the form of tandemly-linked units, therefore, is stably expressed from transformed cells and human insulin can be readily formed from the expressed product.

In summary of this disclosure, the present invention provides a procedure for expressing polypeptides from organisms, which involves providing a DNA fragment containing a multiple number of oligonucleotide sequences each coding for a desired polypeptide product and tandemly-linked by oligonucleotides coding for easily-cleavable amino acid groupings, forming the fragment into a recombinant plasmid and transforming an organism by the plasmid.

## Claims

1. A DNA fragment composed of repeating units coding for a desired product of known amino acid sequence tandemly-linked by oligonucleotides coding for a selectively cleavable amino acid sequence and having no codons for operative combinations of stop and start signals between the ends of said fragment, whereby the fragment is expressible in a suitable vehicle to produce a polypeptide composed of tandemly-linked repeating units of the said desired product.

2. A fragment as claimed in Claim 1 in a replicable cloning vehicle capable of expressing the fragment in a microbial organism.

3. A fragment as claimed in Claim 1 or Claim 2, wherein said selectively-cleavable amino acid sequence includes an aspartyl-proline bond so as to be selectively-cleavable at that bond by acids.

4. A fragment as claimed in Claim 1 or Claim 2 characterised in that the desired product is human proinsulin.

5. A fragment as claimed in any of Claims 1, 2 or 4, characterised by from 2 to 6 repeating units each coding for human proinsulin and being tandemly-linked through olignucleotides coding for selectively cleavable amino acid sequences.

6. A fragment as claimed in any of Claims 1, 2, 4 or 5, wherein said selectively-cleavable amino acid sequence includes a methionine residue so as to be selectively cleavable at that residue by cyanogen bromide.

7. A recombinant plasmid, comprising a plasmid vector and an expressible DNA fragment ligated thereto, characterised in that the DNA fragment is composed of repeating units coding for a desired product of known amino acid sequence tendemly-linked by oligonucleotides coding for a selectively cleavable amino acid sequence, and having no codons for operative combinations of stop and start signals between the ends of said fragment.

8. A plasmid as claimed in Claim 7 wherein said encoded selectively-cleavable amino acid sequence includes a methionine residue so as to be selectively cleavable at that residue by cyanogen bromide.

9. A plasmid claimed in Claim 7 characterised in that the desired product is human proinsulin, the DNA fragment is joined to the 3'-side of a lac operon fragment encoding the first approximately 80 amino acids of β-galactosidase, and the fragment contains from 2 to 5 tandemly-linked oligonucleotide sequences coding for human proinsulin.

10. A plasmid as claimed in Claim 7 characterised in that the desired product is human proinsulin, the DNA fragment is directly linked to a fragment of the cloning vector containing the Tac promoter followed by a bacterial Shine-Dalgarno sequence, and the fragment contains from 3 to 6 tandemly-linked oligonucleotide sequences coding for human proinsulin.

11. A plasmid as claimed in any of Claims 7, 9 or 10 wherein said encoded selectively-cleavable amino acid sequence includes a methionine residue so as to be selectively cleavable at that residue by cyanogen bromide.

12. A microorganism which has been transformed by the plasmid claimed in any one of Claims 7 to 11 for expression of said DNA fragment therefrom.

13. A method of producing polypeptides by microorganisms, characterised by:

(a) cleaving a vector at a predetermined restriction site;

(b) preparing an insert for the cleaved vector including a DNA fragment which has multiple copies of the gene for the polypeptide separated by oligonucleotide sequences coding for selectively cleavable amino acid sequences, and which has no codons for operative combinations of stop and start signals between the ends of said fragment;

(c) ligating the insert to the cleaved vector to form a recombinant plasmid containing the insert;

(d) transforming a microorganism with the recombinant plasmid.

(e) growing said microorganism in a culture medium to effect expression of a DNA fragment containing said multiple copies of the coding sequence for the polypeptide;

(f) isolating from the microorganism a fraction constituting a polymer composed of the multiple linked copies of the polypeptide; and

(g) cleaving the isolated multiple copy polypeptide to single polypeptides.

14. A method as claimed in Claim 13, characterised in that step (f) includes sonic disruption of cells of the microorganism separated from the culture medium.

15. A method of producing polypeptides from a microorganism as claimed in Claim 12, characterised by growing said microorganism in a culture medium to effect expression of a DNA fragment containing said multiple copies of the coding sequence for the polypeptide and to produce a polymer composed of multiple linked copies of the polypeptide; and cleaving the polymer to produce said polypeptide.

16. A method as claimed in any one of Claims 13 to 15 characterised in that the polypeptide is human proinsulin.

17. A method as claimed in Claim 16, characterised in that the human proinsulin is converted into human insulin.

**Patentansprüche**

1. DNA-Fragment, das aus wiederholten Einheiten, die für ein gewünschtes Produkt mit bekannter Aminosäuresequenz kodieren und Tandem-artig durch Oligonukleotide verbunden sind, zusammengesetzt ist, wobei die Oligonukleotide für eine selektiv spaltbare Aminosäuresequenz kodieren und keine Kodons für eine wirksame Kombination von Stopp- und Startsignalen zwischen den Enden des Fragmentes haben, wodurch das Fragment in einem geeigneten Vehikel exprimierbar ist, um ein Polypeptid zu erzeugen, das aus Tandem-artig verbundenen wiederholten Einheiten des gewünschten Produktes zusammengesetzt ist.

2. Fragment nach Anspruch 1 in einem replizierbaren Klonierungsvehikel, das zur Expression des Fragmentes in einem mikrobiellen Organismus fähig ist.

3. Fragment nach Anspruch 1 oder 2, dadurch gekennzeichnet, daß die selektiv spaltbare Aminosäuresequenz eine Aspartyl-Prolinbindung einschließt, um an dieser Bindung durch Säuren selektiv spaltbar zu sein.

4. Fragment nach Anspruch 1 oder 2, dadurch gekennzeichnet, daß das gewünschte Produkt humanes Proinsulin ist.

5. Fragment nach einem der Ansprüche 1, 2 oder 4, gekennzeichnet durch zwei bis sechs wiederholte Einheiten, die jeweils für humanes Proinsulin kodieren und Tandem-artig durch Oligonukleotide verbunden sind, die für selektiv spaltbare Aminosäuresequenzen kodieren.

6. Fragment nach einem der Ansprüche 1, 2, 4 oder 5, dadurch gekennzeichnet, daß die selektiv spaltbare Aminosäuresequenz einen Methioninrest umfaßt, um so an diesem Rest selektiv durch Bromcyan spaltbar zu sein.

7. Rekombinantes Plasmid, umfassend einen Plasmidvektor und ein damit ligiertes exprimierbares DNA-Fragment, dadurch gekennzeichnet, daß das DNA-Fragment aus wiederholten Einheiten zusammengesetzt ist, die für ein gewünschtes Produkt bekannter Aminosäuresequenz kodieren und Tandem-artig durch Oligonukleotide verbunden sind, wobei die Oligonukleotide für eine selektiv spaltbare Aminosäuresequenz kodieren und keine Kodons für eine wirksame Kombination von Stopp- und Startsignalen zwischen den Enden des Fragmentes haben.

8. Plasmid nach Anspruch 7, dadurch gekennzeichnet, daß die kodierte selektiv spaltbare Aminosäuresequenz einen Methioninrest umfaßt, um so an diesem Rest selektiv durch Bromcyan spaltbar zu sein.

9. Plasmid nach Anspruch 7, dadurch gekennzeichnet, daß das gewünschte Produkt humanes Proinsulin ist, das DNA-Fragment mit dem 3'-Ende eines Lac-Operon-Fragmentes verbunden ist, das die ersten ungefähr 80 Aminosäuren der ß-Galactosidase kodiert, und daß das Fragment 2—5 Tandem-artig verbundene Oligonukleotidsequenzen, die für humanes Proinsulin kodieren, enthält.

10. Plasmid nach Anspruch 7, dadurch gekennzeichnet, daß das gewünschte Produkt humanes Proinsulin ist, das DNA-Fragment direkt an ein Fragment des Klonierungsvektors gebunden ist, das den Tac-Promotor enthält, gefolgt von einer bakteriellen Shine-Dalgarno-Sequenz, und daß das Fragment 3—6 Tandem-artig verbundene Oligonukleotidsequenzen enthält, die für humanes Proinsulin kodieren.

11. Plasmid nach einem der Ansprüche 7, 9 oder 10, dadurch gekennzeichnet, daß die kodierte selektiv spaltbare Aminosäuresequenz einen Methioninrest umfaßt, um so an diesem Rest selektiv durch Bromycan spaltbar zu sein.

12. Mikroorganismus, der zur Expression des DNA-Fragmentes mit dem Plasmid nach einem der Ansprüche 7—11 transformiert ist.

13. Verfahren zum Herstellung von Polypeptiden durch Mikroorganismen, gekennzeichnet durch:

(a) Spalten eines Vektors an einer vorbestimmten Restriktionsschnittstelle;

(b) Herstellen eines Inserts für den gespaltenen Vektor einschließlich eines DNA-Fragmentes, das multiple Kopien des Genes für das Polypeptid hat, die durch Oligonukleotidsequenzen getrennt sind, die für selektiv spaltbare Aminosäuresquenzen kodieren, und keine Kodons für die wirksame Verbindung von Stopp- und Startsignalen zwischen den Enden des Fragmentes aufweist;

(c) Ligieren des Inserts mit dem gespaltenen Vektor, um ein das Insert enthaltendes rekombinantes Plasmid zu bilden;

(d) Transformieren eines Mikroorganismus mit dem rekombinanten Plasmid;

(e) Wachsenlassen des Mikroorganismus in einem Kulturmedium, um die Expression eines DNA-Fragmentes, das die multiplen Kopien der kodierenden Sequenz für das Polypeptid enthält, zu bewirken;

(f) Isolieren einer Fraktion aus dem Mikroorganismus, die das Polymer konstituiert und aus multiplen verbundenen Kopien des Polypeptides zusammengesetzt ist; und

(g) Spalten des isolierten Polypeptids mit multiplen Kopien zu einzelnen Polypeptiden.

14. Verfahren nach Anspruch 13, dadurch gekennzeichnet, daß der Schritt (f) ein Aufbrechen der Zellen durch Beschallen des aus dem Kulturmedium abgetrennten Mikroorganismus umfaßt.

15. Verfahren zum Herstellen von Polypeptiden aus einem Mikroorganismus nach Anspruch 12, gekennzeichnet durch Wachsenlassen des Mikroorganismus in einem Kulturmedium, um die Expression eines mehrere Kopien der für das Polypeptid kodierenden Sequenz enthaltenden DNA-Fragmentes zu bewirken und ein Polymer zu erzeugen, das aus multiplen verbundenen Kopien des Polypeptids besteht; und Spalten des Polymers, um das Polypeptid zu erzeugen.

16. Verfahren nach einem der Ansprüche 13 bis 15, dadurch gekennzeichnet, daß das Polypeptid humanes Proinsulin ist.

17. Verfahren nach Anspruch 16, dadurch gekennzeichnet, daß das humane Proinsulin in humanes Insulin umgewandelt wird.

**Revendications**

1. Fragment d'ADN composé de motifs codant pour un produit désiré de séquence d'acides aminés connue liés en tandem par des oligonucléotides codant pour une séquence d'acides aminés clivable sélectivement et n'ayant pas de codons pour des combinaisons opératoires de signaux d'arrêt et de départ entre les extrémités dudit fragment, ce qui fait que le fragment peut être exprimé dans un véhicule approprié pour produire un polypeptide composé de motifs liés en tandem dudit produit désiré.

2. Fragment selon la revendication 1 dans un véhicule de colonage réplicable capable d'exprimer le fragment dans un organisme microbien.

3. Fragment selon la revendication 1 ou la revendication 2, dans lequel ladite séquence d'acides aminés clivable sélectivement comprend une liaison aspartylproline de manière à être sélectivement clivable à cette liaison par des acides.

4. Fragment selon la revendication 1 ou la revendication 2, caractérisé en ce que le produit désiré est la proinsuline humaine.

5. Fragment selon l'une quelconque des revendications 1, 2, ou 4, caractérisé par de 2 à 6 motifs codant chacun pour la proinsuline humaine et liés en tandem par des oligonucléotides codant pour des séquences d'acides aminés sélectivement clivables.

6. Fragment selon l'une quelconque des revendications 1, 2, 4 ou 5, dans lequel ladite séquence d'acides aminés sélectivement clivable comprend un reste méthionine de manière à être sélectivement clivable à ce reste par du bromure de cyanogène.

7. Plasmide recombinant comprenant un vecteur plasmidique et un fragment d'ADN exprimable qui y est lié, caractérisé en ce que le fragment d'ADN est composé de motifs codant pour un produit désiré de séquence d'acides aminés connu liés en tandem par des oligonucléotides codant pour une séquence d'acides aminés sélectivement clivable, et n'ayant pas de codons pour des combinaisons opératoires de signaux d'arrêt et de départ entre les extrémités dudit fragment.

8. Plasmide selon la revendication 7 dans lequel ladite séquence d'acides aminés sélectivement clivable encodée comprend un reste méthionine de manière à être sélectivement clivable à ce reste par du brumure de cyanogène.

9. Plasmide selon la revendication 7 caractérisé en ce que le produit désiré est la proinsuline humaine, en ce que le fragment d'ADN est réuni au côté 3' d'un fragment d'opéron lac encodant les environ 80 premiers acides aminés de la β-galactosidase, et le fragment contient de 2 à 5 séquences d'oligonucléotides codant pour la proinsuline humaine liées en tandem.

10. Plasmide selon la revendication 7 caractérisé en ce que le produit désiré est la proinsuline humaine, le fragment d'ADN est directement lié à un fragment du vecteur de clonage contenant le promoteur Tac suivi par une séquence bactérienne de Shine-Dalgarno, et le fragment contient de 3 à 6 séquences d'oligonucléotides codant pour la proinsuline humaine liées en tandem.

11. Plasmide selon l'une quelconque des revendications 7, 9 ou 10 dans lequel ladite séquence d'acides aminés sélectivement clivable encodée comprend un reste méthionine de manière à être sélectivement clivable à ce reste par du bromure de cyanogène.

12. Microorganisme qui a été transformé par le plasmide revendiqué dans l'une quelconque des revendications 7 à 11 pour l'expression dudit fragment d'ADN.

13. Procédé de production de polypeptides par des microorganismes, caractérisé en ce que:

(a) un clive un vecteur en un site de restriction prédéterminé;

(b) on prépare un insert pour le vecteur clivé incluant un fragment d'ADN qui à des exemplaires multiples du gène pour le polypeptide séparé par des séquences d'oligonucléotides codant pour des séquences d'acides aminés sélectivement clivables, et qui n'a pas de codons pour des combinaisons opératoires de signaux d'arrêt et de départ entre les extrémités dudit fragment;

(c) on lie l'insert au vecteur clivé pour former un plasmide recombinant contenant l'insert;

(d) on transforme un microorganisme avec le plasmide recombinant;

(e) on cultive ledit microorganisme dans un milieu de culture pour effectuer l'expression d'un fragment d'ADN contenant lesdits exemplaires multiples de la séquence de codage pour le polypeptide;

(f) on isole du microorganisme une fraction constituant un polymère composé de multiples exemplaires liés au polypeptide; et

(g) on clive le polypeptide à exemplaires multiples isolé en polypeptides uniques.

14. Procédé selon la revendication 13, caractérisé en ce que l'étape (f) comprend une rupture par ultrasons des cellules du microorganisme séparées du mileu de culture.

15. Procédé de production de polypeptides à partir d'un microorganisme selon la revendication 12, caractérisé en ce qu'on cultive ledit microorganisme dans un milieu de culture pour effectuer l'expression d'un fragment d'ADN contenant lesdits exemplaires multiples de la séquence de codage pour le polypeptide et pour produire un polymère composé de multiples exemplaires liés au polypeptide; et en ce qu'on clive le polymère pour produire ledit polypeptide.

16. Procédé selon l'une quelconque des revendications 13 à 15 caractérisé en ce que le polypeptide est la proinsuline humaine.

17. Procédé selon la revendication 16, caractérisé en ce que la proinsuline humaine est transformée en insuline humaine.

## FIG.1.

EP 0 163 406 B1

OLIGONUCLEOTIDES

EcoRI  PI  BamHI

pAT/PIR

EcoRI
BamHI

EcoRI          BamHI     BamHI

SfaNI

CAP
SfaNI

(oh)AATTC
G          GGAGp

EcoRI PI
Tac

pTac/PI

EcoRI
BamHI

EcoRI
Tac          BamHI

PTac

Ligation

EcoRI(PI)
Tac          PI
pTac/2PI     BamHI

CCTCT———A———C          A———B———GTTAA
kinase
$^{32}$P-γ-ATP

Anneal

$^{32}$PCCTCT——————————C TTAA (oh)
A——————————G

T4 DNA Ligase

EcoRI     PI(Analog)     (RI)
(oh)AATTC——————$^{32}$P——C TTAA(oh)
G——————P——G
Kinase
ATP

T4 DNA Ligase
EcoRI
BamHI

EcoRI     BamHI          BamHI
PI(Analog)  (RI)  PI

Lac          EcoRI
z          PI
plac239/     BamHI
PI

EcoRI
BamHI
BamHI

EcoRI
Lac     z
Plac239

Ligation

EcoRI (PI)
Lac
z
plac239/     PI
2PI          BamHI

FIG. 2.

Hind III

BamHI-Hind III
from plac 504 PI

plac 239-3PI

8·0 Kbp

lac promoter

β galactoside leader
of 239 bp

Sal I

[EcoRI]

BamHI

[EcoRI]/BamH1
containing PI
genes.

BamH1                    [EcoRI]

[Eco RI]/Bam H1 segment =

BamH1    [EcoRI]

= PI coding sequence

= amino acid linker
coding sequence

FIG.3.

BamHI-Hind III portion
from plac 504/PI

pTac/5PI
5·5 Kbp

BamHI

95bp

Hind III

EcoRI

Tac promoter from
pDR 540

Eco RI/BamH1
segment
containing PI genes

BamH1

EcoRI

EcoRI/BamH1segment

= PI coding sequence

= amino acid linker
coding sequence

5' AATTCTATGTTTGTCAAT
3'     GATACAAACAGTTAGTCG

FIG.4.

# FIG.5.

FIG.6a.

FIG.6 b.

FIG.7.

plac 504/PI

(1) Digest with MstII.
(2) Fill in sticky ends with dTTP and dGTP

Z
CCTG
GGACT 5'

5' TCAGG
GTCC

Z

Lac

PI
BamHI    EcoRI

(1) Take off 5' T's with mung bean nuclease.
(2) Cut with EcoRI.
(3) Fill in with dATP and dTTP.

Z
CCTG
GGAC

AATTCTATG
TTAAGATAC

PI BamHI

Lac

Blunt end ligate

EcoRI site

Z
CCT GAA TTCT ATG
GGC CTT AAGA TAC

PI

BamHI

Lac

plac 239 / PI

(1) Cut with EcoRI.
(2) Digest tails with mung bean nuclease.
(3) Blunt end ligate.

Pro  Ala  Met
CCT  GCT  ATG
GGC  CGA  TAC

PI

BamH1

Z

Lac

plac 239-PI

FIG.8.

1 2 3 4 5 M

FIG. 9.

FIG.10.

A B M

FIG.11.